# EUROPEAN PATENT SPECIFICATION

(11) **EP 3 974 835 B1**
(45) Date of publication and mention of the grant of the patent: **26.02.2025**
(21) Application number: 20835450.6
(22) Date of filing: 03.07.2020
(51) Int. Cl.: G01N 33/68, G01N 33/574, G01N 33/533, G01N 33/543, G01N 33/50

(54) **METHOD FOR DETECTING TUMOR CELL SURFACE MARKER MOLECULE PD-L1**
VERFAHREN ZUM NACHWEIS DES TUMORZELLOBERFLÄCHENMARKERMOLEKÜLS PD-L1
MÉTHODE DE DÉTECTION DE MOLÉCULE MARQUEUR DE SURFACE DE CELLULE TUMORALE PD-L1

(30) Priority: 04.07.2019 CN 201910601477
(43) Date of publication of application: 30.03.2022
(73) Proprietor: Hemosmart Medical Technology Ltd., Yushan Town Kunshan Jiangsu 215300 (CN)
(72) Inventor: YAN, Jing, Kunshan, Jiangsu 215300 (CN)
(74) Representative: Fidal Innovation
(86) International application number: PCT/CN2020/100189
(87) International publication number: WO 2021/000949

(56) References cited:
- EP-A1- 3 364 190
- WO-A1-2007/134191
- WO-A1-2018/017604
- CN-A- 106 198 984
- CN-A- 106 198 984
- CN-A- 107 338 184
- CN-A- 107 338 184
- CN-A- 107 561 265
- CN-A- 108 507 992
- CN-A- 109 874 316
- CN-A- 110 361 536
- US-A1- 2010 233 694
- JANNING MELANIE ET AL: "Determination of PD-L1 Expression in Circulating Tumor Cells of NSCLC Patients and Correlation with Response to PD-1/PD-L1 Inhibitors", CANCERS, vol. 11, no. 6, 17 June 2019 (2019-06-17), pages 835, XP055928503, Retrieved from the Internet <URL:https://www.ncbi.nlm.nih.gov/pmc/articles/PMC6627043/pdf/cancers-11-00835.pdf> [retrieved on 20220613], DOI: 10.3390/cancers11060835
- WANG YANG ET AL: "PD-L1 Expression in Circulating Tumor Cells Increases during Radio(chemo)therapy and Indicates Poor Prognosis in Non-small Cell Lung Cancer", SCIENTIFIC REPORTS, vol. 9, no. 1, 24 January 2019 (2019-01-24), XP093048197, DOI: 10.1038/s41598-018-36096-7

## Description

### Technical Field

The present invention relates to the biotechnology field, in particular to a method for detecting PD-L1 which is a tumor cell surface marker molecule.

### Background

Programmed death protein-1 (PD-1) is the main immune checkpoint receptor, and by binding to its ligand, Programmed Death Ligand-1 (PD-L1), it can down-regulate the effector function of T cells, thereby helping to maintain tolerance to tumor cells. There are currently three main inhibitors for PD-1 and PD-L1 on the market, namely pembrolizumab (trade name: Keytruda), Nibolumab (trade name: Opdivo) and Atezolizumab (trade name: Tecentriq), which can be used for the treatment of various cancers such as melanoma, non-small cells lung cancer and bladder cancer. However, not all patients can benefit from the treatment of PD-1/PD-L1 inhibitors, PD-1/PD-L1 inhibitors are currently only able to produce lasting tumor control effects in a small number of cancer patients. Therefore, detecting whether the patient has positive expression of PD-L1 can effectively help the patient choose the appropriate medicine for treatment. At present, the detection methods of PD-1/PD-L1 are mainly based on the detection of cellular protein levels, and in clinical practice, immunohistochemical methods are mainly used, and tumor tissues obtained after surgery or puncture are used for section staining. The results of immunohistochemistry are closely related to the experience of the pathologist. Therefore, a new non-invasive evaluation method and standard, and a relatively stable PD-L1 detection method are urgently needed.

Circulating tumor cells fall off from the primary tumor, enter the blood circulation, settle in remote organs or primary organs, and form metastasis foci. As a hot field of liquid biopsy, circulating tumor cell (CTC) detection has gradually emerged in the clinical manifestations such as tumor diagnosis, treatment and monitoring, and is currently the most promising non-invasive tumor diagnosis and real-time efficacy monitoring method, and has extremely significant clinical application value. Therefore, it will be of great significance if a method for detecting PD-L1 on the surface of tumor cells such as circulating tumor cells can be provided.

For example, Chinese patent CN201810312287.X discloses a method for detecting circulating tumor cell surface marker molecule PD-L1, which comprises the following steps: (1) treating whole blood with the red cell lysing solution to separate nucleated cells and fixing them with formaldehyde; (2) first positive screening by tumor immunofluorescence marker cytokeratin antibody anti-CK, incubating all cells with PD-L1 antibody, then incubating all cells with PD-L1 secondary antibody labeled with FITC fluorophore, and then labeling all cells with nuclear fluorescent dye DAPI; (3) using high-throughput multicolor imaging analysis, selecting CY5, FITC and DAPI filters, observing the fluorescence color of the channel surface, to finally achieve the detection of the circulating tumor cell surface marker molecule PD-L1. This detection method uses whole blood to be processed with red cell lysing solution to separate nucleated cells, but fails to directly separate CTC cells, where the background cells are complex and it is difficult to ensure the accuracy of the detection.

Another example is the Chinese patent CN201610705258.0 discloses a method for detecting the PDL1 gene of circulating tumor cells in the peripheral blood of patients with non-small cell lung cancer, which comprises the following steps: (1) membrane filtering the peripheral blood samples from patients with non-small cell lung cancer, to obtain circulating tumor cells in the peripheral blood; (2) fixing the filter membrane obtained in step 1; (3) detecting the filter membrane obtained in step 2 to determine the expression of PD-L1. This detection method cannot accurately obtain CTCs with PD-L1 expression only by relying on these three steps, and must be combined with HE staining and expert reading to accurately identify CTCs. On the one hand, such steps are cumbersome and complicated; on the other hand, expert reading not only has a strong subjectivity to affect the judgment result, but also has strong professionalism to make it difficult to be promoted and used.

Other reference can be found in CN106198984A, JANNING MELANIE ET AL: "Determination of PD-L1 Expression in Circulating Tumor Cells of NSCLC Patients and Correlation with Response to PD-1/PD-L1 Inhibitors", CANCERS, vol. 11 , no. 6, 17 June 2019 (2019-06-17), doi : 1 0.3390/cancers11 060835, CN107338184A, WANG YANG ET AL: PD-L1 Expression in Circulating Tumor Cells Increases during Radio(chemo)therapy and Indicates Poor Prognosis in Non-small Cell Lung Cancer", SCIENTIFIC REPORTS, vol. 9, no. 1, 24 January 2019(2019-01-24), doi : 10.1 038/s41598-018-36096-7, CN109874316A and US2010233694A1.

### Summary

The present invention is aimed to provide a novel method for detecting a tumor cell surface marker molecule PD-L1, which improves detection accuracy and is easier in terms of detection operation.

The invention is set out in the appended set of claims.

Here, the term "room temperature" refers to 20 ~ 25 °C.

Due to the use of the above solutions, the present invention has the following advantages over the prior art:
using a capture screen to capture tumor cells in body fluids (such as blood, urine or peritoneal fluid) and then incubating and detecting through PD-L1 and other antibodies, effectively avoids the effects of various other cells, cytokines and proteins in body fluid on the incubation and detection of PD-L1 and other antibodies, which improves detection accuracy and reliability, and has better specificity; and the capture screen is directly used as the carrier for incubating and detecting the captured and separated tumor cells, which is simple and convenient.

### Brief Description of the Drawings

For more clearly explaining the technical solutions in the embodiments of the present invention, the accompanying drawings used to describe the embodiments are simply introduced in the following. Apparently, the below described drawings merely show a part of the embodiments of the present invention, and those skilled in the art can obtain other drawings according to the accompanying drawings without creative work.
Fig. 1a and Fig. 1b are schematic diagrams of detecting positive and negative expression of CK in tumor cells by a FITC filter;
Fig. 2a and Fig. 2b are schematic diagrams of detecting positive and negative expression of PD-L1 in tumor cells by a PD-L1 (primary antibody)-Alexa Fluor 647 (secondary antibody) filter;
Fig. 3a and Fig. 3b are schematic diagrams of detecting negative expression of CK45 in tumor cells by a PE filter;
Fig. 4a and Fig. 4b are schematic diagrams of detecting whether tumor cells are nucleated cells by a DAPI filter;
Fig. 5a and Fig. 5b are schematic diagrams of detecting the positive and negative expression of PD-L1 in tumor cells by combining four kinds of filters.

### Detailed Description of Exemplary Embodiments

In the following, the preferable embodiments of the present invention are explained in detail combining with the accompanying drawings so that the advantages and features of the present invention can be easily understood by the skilled persons in the art. It should be noted that the explanation on these implementations is to help understanding of the present invention, and is not intended to limit the present invention.

### Embodiment 1

This embodiment detected the tumor cell surface marker molecule PD-L1, which was specifically carried out as follows.

### (1) Providing the capture screen

The capture screen comprises a stainless-steel body and a protective layer covering the surface of the stainless-steel body. The material of the protective layer is gold or gold alloy (such as, AuPd), and the EpCAM antibodies are arranged on the protective layer. The protective layer is an AuPd layer deposited on the stainless-steel body by magnetron sputtering or electrochemical methods. The EpCAM antibodies was attached to the protective layer via Traut's reagent, and thiolate molecules with biotin-avidin can be replace the Traut's reagent.

The specific preparation process of the above-mentioned capture screen was as follows.

Selection of screen: gold-coated stainless-steel screen: 51µm holes were chosen and magnetron sputtering was used to coat AuPd, the size of the screen was 2 × 2 mm². Pre-functionalization: various cleaning methods were used to prepare the screen before functionalization, including high-pressure steam sterilization, oxygen plasma cleaning, and ultrasonic cleaning in a variety of solutions, including piranha solutions, ammonia-hydrogen peroxide mixing liquid. For example, the screen was ultrasonic treated in a detergent for 15 minutes, rinsed, ultrasonic treated in 70% ~ 99% ethanol solution for 15 minutes, and rinsed with high purity water for 5 minutes.

EpCAM antibodies: 2 ~ 10 µl of EpCAM antibodies were taken and freezed, and then used to prepare a reaction mixture (i.e., EpCAM antibodies + PBS with EDTA).

Traut's Reagent: it was freezed quickly after purchase. The volume ratio of Traut's reagent and EpCAM antibodies was 10 ~ 20: 1. Other methods, such as thiolate molecules with biotin-avidin may replace the Traut's reagent to connect to the screen to form capture screen.

Incubation time of Traut's reagent with antibody: the best reaction time is 1 hour.

Incubation of the screen in the above solution: the screen with the above incubated antibody-containing Traut's reagent was incubated at 4°C, room temperature or 37 °C for 10 minutes to within 12 hours, so that the EpCAM antibodies were connected to the screen.

(2) A patient's peripheral blood was collected, and red blood cell lysing solution or lymphocyte separation solution was used to separate nucleated cells;
the nucleated cells flowed through the capture screen once or repeatedly, during this process, the tumor cells were bound to the EpCAM antibodies on the capture screen, thereby being captured;
the capture screen was washed with a cell washing solution, and other impurities or cells that did not specifically bind to the EpCAM antibodies on the capture screen were eluted and removed, and only tumor cells that specifically binded to the EpCAM antibodies were left on the capture screen.

(3) The capture screen bound with the tumor cells was placed in 500 µL of 4% paraformaldehyde solution, fixed at room temperature for 20 min, and washed with phosphate buffer for 2 times.;

(4) 200 µL of PD-L1 primary antibody solution was added (the volume ratio of antibody stock solution and PBS was 1: 200), and the cells fixed on the capture screen were incubated at 25 °C for 60 min, and then washed with 200 µL of phosphate buffer for 2 times.

(5) 200 µL of PD-L1 secondary antibody solution labeled with fluorophore AlexaFluor 647 was added (the volume ratio of antibody stock solution and PBS was 1: 100), and the cells were incubated at 25 °C for 60 min, and then washed with phosphate buffer for 2 times.

(6) 200 µL of pan-CK-AlexaFluor 488 primary antibody solution was added (the volume ratio of antibody stock solution and PBS was 1: 100), and the cells fixed on the capture screen were incubated at 25 °C for 60 min, and then washed with 200 µL of phosphate buffer for 2 times.

(7) 200 µL of CD45 primary antibody solution was added (the volume ratio of antibody stock solution and PBS was 1: 200), and the cells fixed on the capture screen were incubated at 25 °C for 60 min, and then washed with 200 µL of phosphate buffer for 2 times.

(8) 200 µL of CD45 secondary antibody solution labeled with fluorophore AlexaFluor 568 was added (the volume ratio of antibody stock solution and PBS was 1: 100), and the cells were incubated at 25 °C for 60 min, and then washed with phosphate buffer for 2 times.

(9) All the cells on the capture screen were labeled with 200 µL of nuclear fluorescent dye DAPI.

(10) Through high-throughput multicolor imaging analysis using filters of CY5, FITC, PE and DAPI, the fluorescence color of each channel was observed to detect the tumor cell surface marker molecule PD-L1.

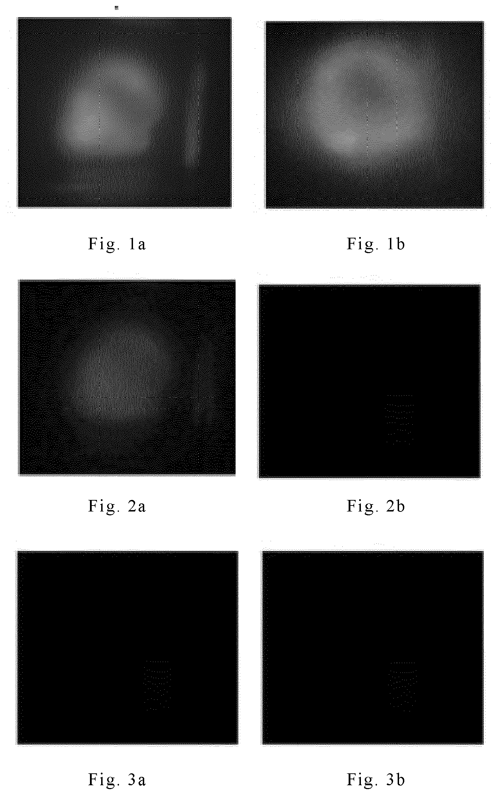

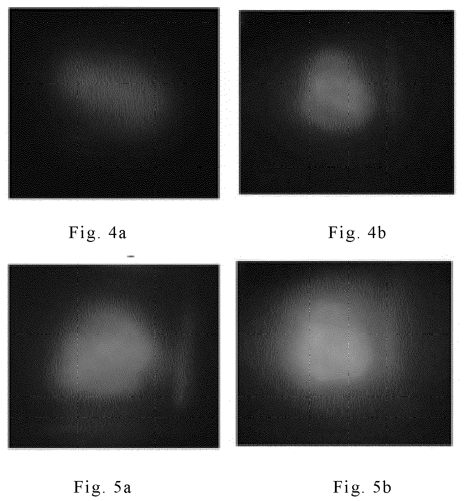

The results of the detection of the positive and negative expression of the tumor cell surface marker molecule PD-L1 by a pan-CK filter (green), a PD-L1 (primary antibody)-Alexa Fluor 647 (secondary antibody) filter (red), a CD45 (primary antibody)-Alexa Fluor 568 (secondary antibody) filter (red ), a DAPI filter (blue) and the combination of the four are shown in Fig. 1a to Fig. 5a and Fig. 1b to Fig. 5b, respectively.

### Experiments on the influence of incubation sequence on detection accuracy

**1. Test sample:** collecting 4 mL of healthy human peripheral blood, adding about 200 cells of the transfected NCI-H226 reference cell line to it, using lymphocyte separation solution to separate nucleated cells PBMC, and using the nucleated cells PBMC as the test sample.

### 2. Experimental process

**Experimental group:** performing detection according to the detection method of this application (the incubation sequence is: PD-L1+ secondary antibody, pan-CK, CD45+ secondary antibody, DAPI staining), the specific process was as follows.
(1) Preparing the capture screen: referring to the corresponding step (1) in Embodiment 1 for the preparation of the capture screen.
(2) Capturing cells: collecting 4 mL of healthy human peripheral blood, adding about 200 cells of the transfected NCI-H226 reference cell line to it, and using lymphocyte separation solution to separate nucleated cells PBMC. The nucleated cells PBMC flowed through the capture screen once or repeatedly, during this process, the tumor cells were bound to the EpCAM antibodies on the capture screen, MCF-7 cells were captured, and finally, the capture screen was washed with a cell washing solution, to elute and remove other impurities or cells that did not specifically bind to the EpCAM antibodies on the capture screen, and only tumor cells that specifically binded to the EpCAM antibodies were left on the capture screen.
(3) Fixing the cells: the capture screen bound with the tumor cells was placed in 500 µL of 4% paraformaldehyde solution, fixed at room temperature for 20 min, and washed with phosphate buffer for 2 times.
(4) 200 µL of PD-L1 primary antibody solution was added (the volume ratio of antibody stock solution and PBS was 1: 200), and the cells fixed on the capture screen were incubated at 25 °C for 60 min, and then washed with 200 µL of phosphate buffer for 2 times.
(5) 200 µL of PD-L1 secondary antibody solution labeled with fluorophore AlexaFluor 647 was added (the volume ratio of antibody stock solution and PBS was 1: 100), and the cells were incubated at 25 °C for 60 min, and then washed with phosphate buffer for 2 times.
(6) 200 µL of pan-CK-AlexaFluor 488 primary antibody solution was added (the volume ratio of antibody stock solution and PBS was 1: 100), and the cells fixed on the capture screen were incubated at 25 °C for 60 min, and then washed with 200 µL of phosphate buffer for 2 times.
(7) 200 µL of CD45 primary antibody solution was added (the volume ratio of antibody stock solution and PBS was 1: 200), and the cells fixed on the capture screen were incubated at 25 °C for 60 min, and then washed with 200 µL of phosphate buffer for 2 times.
(8) 200 µL of CD45 secondary antibody solution labeled with fluorophore AlexaFluor 568 was added (the volume ratio of antibody stock solution and PBS was 1: 100), and the cells were incubated at 25 °C for 60 min, and then washed with phosphate buffer for 2 times.
(9) All the cells on the capture screen were labeled with 200 µL of nuclear fluorescent dye DAPI.
(10) Through high-throughput multicolor imaging analysis using filters of CY5, FITC, PE and DAPI, the fluorescence color of each channel was observed.

**Control group 1:** On basis of the detection method of this application, the incubation sequence was changed (the sequence was CD45+ secondary antibody, PD-L1+ secondary antibody, pan-CK, DAPI staining), the specific process was as follows.
(1) Providing the capture screen: same as the experimental group;
(2) Capturing cells: same as the experimental group;
(3) Fixing cells: same as the experimental group;
(4) 200 µL of CD45 primary antibody solution was added (the volume ratio of antibody stock solution and PBS was 1: 200), and the cells fixed on the capture screen were incubated at 25 °C for 60 min, and then washed with 200 µL of phosphate buffer for 2 times.
(5) 200 µL of CD45 secondary antibody solution labeled with fluorophore AlexaFluor 568 was added (the volume ratio of antibody stock solution and PBS was 1: 100), and the cells were incubated at 25 °C for 60 min, and then washed with phosphate buffer for 2 times.
(6) 200 µL of PD-L1 primary antibody solution was added (the volume ratio of antibody stock solution and PBS was 1: 200), and the cells fixed on the capture screen were incubated at 25 °C for 60 min, and then washed with 200 µL of phosphate buffer for 2 times.
(7) 200 µL of PD-L1 secondary antibody solution labeled with fluorophore AlexaFluor 647 was added (the volume ratio of antibody stock solution and PBS was 1: 100), and the cells were incubated at 25 °C for 60 min, and then washed with phosphate buffer for 2 times.
(8) 200 µL of pan-CK-AlexaFluor 488 primary antibody solution was added (the volume ratio of antibody stock solution and PBS was 1: 100), and the cells fixed on the capture screen were incubated at 25 °C for 60 min, and then washed with 200 µL of phosphate buffer for 2 times.
(9) All the cells on the capture screen were labeled with 200 µL of nuclear fluorescent dye DAPI.
(10) Through high-throughput multicolor imaging analysis using filters of CY5, FITC, PE and DAPI, the fluorescence color of each channel was observed to detect the tumor cell surface marker molecule PD-L1. This procedure was also the same as in the experimental group.

**Control group 2:** On basis of the detection method of this application, the incubation sequence was changed (the sequence was CD45+ secondary antibody, pan-CK, PD-L1+ secondary antibody, DAPI staining), the specific process was as follows:
(1) Providing the capture screen: same as the experimental group;
(2) Capturing cells: same as the experimental group;
(3) Fixing cells: same as the experimental group;
(4) 200 µL of CD45 primary antibody solution was added (the volume ratio of antibody stock solution and PBS was 1: 200), and the cells on the capture screen were incubated at 25 °C for 60 min, and then washed with 200 µL of phosphate buffer for 2 times.
(5) 200 µL of CD45 secondary antibody solution labeled with fluorophore AlexaFluor 568 was added (the volume ratio of antibody stock solution and PBS was 1: 100), and the cells were incubated at 25 °C for 60 min, and then washed with phosphate buffer for 2 times.
(6) 200 µL of pan-CK-AlexaFluor 488 primary antibody solution was added (the volume ratio of antibody stock solution and PBS was 1: 100), and the cells on the capture screen were incubated at 25 °C for 60 min, and then washed with 200 µL of phosphate buffer for 2 times.
(7) 200 µL of PD-L1 primary antibody solution was added (the volume ratio of antibody stock solution and PBS was 1: 200), and the cells fixed on the capture screen were incubated at 25 °C for 60 min, and then washed with 200 µL of phosphate buffer for 2 times.
(8) 200 µL of PD-L1 secondary antibody solution labeled with fluorophore AlexaFluor 647 was added (the volume ratio of antibody stock solution and PBS was 1: 100), and the cells were incubated at 25 °C for 60 min, and then washed with phosphate buffer for 2 times.
(9) All the cells on the capture screen were labeled with 200 µL of nuclear fluorescent dye DAPI.
(10) Through high-throughput multicolor imaging analysis using filters of CY5, FITC, PE and DAPI, the fluorescence color of each channel was observed to detect the tumor cell surface marker molecule PD-L1. This procedure was also the same as in the experimental group.

### 3. Results and Analysis

CTC criterion: For the cells in the same position, keep the capture screen sample still, switch the filter of the microscope, observe the fluorescence effect of the cells after staining with different dyes, the sequence to be switches is green fluorescence (CK), blue fluorescence (DAPI), red fluorescence (CD45), and magenta fluorescence (PD-L1). If the cell fluorescence captured on the chip is CK-positive, DAPI-positive, and CD45-negative (which can effectively eliminate false positives), it can be identified as a CTC.

The criterion for PD-L1 expression: after confirming that the cell is a CTC, identify the PD-L1 stained fluorescence at the position with the CTC, and if the CTC has PD-L1 expression (i.e., magenta fluorescence), then the CTC has PD-L1 expression; if there is no magenta fluorescence, the CTC does not have PD-L1 expression.

According to the above criteria, according to the experimental conditions of each group, the detection results are summarized as shown in Table 1.

**Table 1 Fluorescence detection results of experimental group and control groups**

| **Items** | **Staining sequence** | **Total number of CTCs** | **The number of CTCs with PD-L1 expression** | **The ratio of CTCs with PD-L1 expression to the total CTCs** |
|---|---|---|---|---|
| Experime ntal group | PD-L1+secondary antibody, pan-CK, CD45+secondary antibody, DAPI | 60 | 60 | 100% |
| Control group 1 | CD45+secondary antibody, PD-L1+secondary antibody, pan-CK, DAPI | 73 | 62 | 84.9% |
| Control group 2 | CD45+secondary antibody, pan-CK, PD-L1+secondary antibody, DAPI | 51 | 41 | 80.4% |

| | | | | |
|---|---|---|---|---|
| Note: The numbers of CTC cells in the above table corresponds to the numbers of CTC cells in a certain area on the capture screen, not all CTC cells in the test sample; the selected areas before and after the staining in the same group of experiments are the same. | | | | |

From the results in the above table, it can be seen that the proportion of CTCs with PD-L1 expression in the control group 1 and the control group 2 is significantly lower than the result in the experimental group, indicating that some CTCs with PD-L1 expression in control group 1 and control group 2 were not detected. It shows that using the staining sequence in the control group 1 and the control group 2, the fluorescence of PD-L1 of some CTCs is weak, and some do not even have expression of PD-L1, that is, cells with positive PD-L1 expression are not stained. The reason for the analysis to cause such results may be: using the staining sequence in the control group 1 and the control group 2, the AlexaFluor 647-labeled PD-L1 secondary antibody binds to the CD45 antibody which reduces the binding efficiency of the PD-L1 primary antibody and the AlexaFluor 647-labeled secondary antibody, which leads to inaccurate detection of CTCs with PD-L1 expression, and reduces the expression efficiency of PD-L1.

In the embodiment of this application, a capture screen connected with EpCAM antibodies is used to specifically capture tumor cells in nucleated cells by controlling the flow rate of the system through the microfluidics; then to the capture screen that captures tumor cells, a PD-L1 antibody solution is used and combined with the use of three fluorescent antibodies, namely DAPI, CK and CD45, to identify the CTCs with PD-L1 expression. That is to say, the embodiment only needs to use the capture screen to specifically capture the cells, and only perform two steps of incubation and combined immunofluorescence analysis to accurately identify the CTCs with PD-L1 expression.

Through the technical solutions of the embodiments of this application, in addition to the excellent effects brought about by the above-mentioned staining sequence, it also has the following advantages:
1. this application adopts the method of physical + antigen-antibody binding, which can specifically bind tumor cells chemically to the mesh substrate, and control the flow rate of the system through microfluidics to specifically capture tumor cells in nucleated cells and capture thoroughly, it belongs to the microfluidic product, the capture accuracy is high, there will be no missed capturing or capturing too much due to the size of the cells in the filter retention scheme, and there will be no complicated background cells in the final detection process, which ensures the accuracy of detection and identification.
2. The staining method used in this application is an in-situ staining method, in which the capture screen with the tumor cells is used as the carrier, the captured cells do not need to be transferred, it is only necessary to use the PD-L1 antibody solution in situ on the surface of the carrier and use three fluorescent antibodies, namely DAPI, CK, and CD45, in combination, and can accurately identify CTCs with PD-L1 expression, effectively eliminate false positives, such as blood-derived cell interference or non-specific adsorption interference.
   a. The PD-L1 primary antibody solution and the PD-L1 secondary antibody solution labeled with the fluorophore AlexaFluor 647 are first used to label the site of PD-L1 expression in situ;
   b. then the pan-CK-AlexaFluor 488 primary antibody solution is used to label CTCs (circulating tumor cells);
   c. then the CD45 primary antibody solution and the CD45 secondary antibody solution labeled with the fluorophore AlexaFluor 568 are used to label the white blood cells to remove the interference of white blood cells and eliminate false positives;
   d. finally, the nuclear fluorescent dye (DAPI) is used to label the nucleated cells; the combination of the above several fluorescent antibodies can accurately identify CTCs with PD-L1 expression, the results obtained without any of these fluorescent antibodies are inaccurate, and because the total CTCs and the CTCs with PD-L1 expression are labeled at the same time, therefore, the ratio of CTCs with PD-L1 expression to the total CTCs can be obtained.
3. In the embodiment of this application, the capture screen that specifically captures tumor cells is used as a carrier to detect and incubate in situ, the captured tumor cells are fixed in place on the capture screen, and the location of the capture screen can also be kept unchanged, and the microfluidic system is used to make the antibody solutions flow to the position of the capture screen for incubation, even if the antibody solutions flow up and down in the capture screen, it will not cause the captured tumor cells to fall; when performing PD-L1 identification, it only needs to directly place the capture device in the microscope for observation; it simplifies the operation process and reduces the complexity of the operation while ensuring the accuracy of the detection.
4. In the embodiment of the present application, there is no need to remove the capture screen and fix it on other carriers with an adhesive during incubation and detection, which will not affect the captured cells and simplify the operation process.
5. The pan-CK includes many kinds of CK antibodies, and in this application, the pan-CK-AlexaFluor 488 antibody is used as a tumor marker for positive screening, which has high enrichment efficiency and enrichment accuracy, avoids omission of tumor cells in different stages of differentiation, and improves the accuracy of detection.
6. Under normal circumstances, the proportion of false positives in detection samples will be greater than 10%. The incubation and identification method in this application can accurately determine the false positive samples, so that the false positive samples are eliminated from the total number of samples to obtain more accurate identification results.
7. In the embodiment of this application, the tumor cells are complete cells throughout from the initial capture to the subsequent incubation and detection, there is no step of permeabilizing the cells, and the integrity of the cell morphology and structure will not be destroyed.
8. The samples detected in the embodiment of this application are nucleated cells obtained by separating peripheral blood using red blood cell lysing sulution or lymphocyte separation solution, which can further avoid the influence of other cells, cytokines and proteins in the body fluid on the PD-L1 antibody incubation detection, and improve the accuracy and reliability of the detection.
9. The technical solution based on the capture screen and the microfluidic system in this application is easier to realize automation, and the capture screen can be fixed in a specific device, and the antibody solutions can be sucked into the device for incubation, detection and other steps.
10. The detection results obtained by adopting the technical solution in this application can effectively avoid the interference of human subjective factors in the traditional HE staining combined with expert reading methods, with higher accuracy and more popularization.

The detection method of the present application is mainly used for non-diagnostic purposes of PD-L1 detection, but can also be used for diagnostic purposes.

The embodiments described above are only for illustrating the technical concepts and features of the present invention, are preferred embodiments, and are intended to make those skilled in the art being able to understand the present invention and thereby implement it.

## Claims

1. A method for detecting a tumor cell surface marker molecule PD-L1, **characterized in that**, the method comprises the following steps:
providing a capture screen that has antibodies capable of specifically binding to tumor cells, wherein the capture screen comprises a mesh substrate and EpCAM antibodies arranged on the mesh substrate by incubation;
making a sample to be tested flow through the capture screen, such that tumor cells in the sample to be tested bind to the capture screen;
fixing captured tumor cells on the capture screen; and
successively using a PD-L1 primary antibody solution, a PD-L1 secondary antibody solution labeled with a fluorophore AlexaFluor 647, a pan-CK-AlexaFluor 488 primary antibody solution, a CD45 primary antibody solution and a CD45 secondary antibody solution labeled with a fluorophore AlexaFluor 568, to incubate the cells fixed on the capture screen, and then labeling all cells on the capture screen with a nuclear fluorescent dye.

2. The detection method according to claim 1, is **characterized in that**, during incubating, the entire capture screen with fixed tumor cells is used as a carrier for incubation.

3. The detection method according to claim 1 or 2, is **characterized in that**, the cells fixed on the capture screen is incubated as follows:
adding a PD-L1 primary antibody solution, incubating the cells fixed on the capture screen at room temperature for 20 ~ 80 min, and then washing with phosphate buffer;
adding a PD-L1 secondary antibody solution labeled with fluorophore AlexaFluor 647, incubating at room temperature for 20 ~ 80 min, and washing with phosphate buffer;
adding a pan-CK-AlexaFluor 488 primary antibody solution, incubating the cells fixed on the capture screen at room temperature for 20 ~ 80 min, and washing with phosphate buffer;
adding a CD45 primary antibody solution, incubating the cells fixed on the capture screen at room temperature for 20 ~ 80 min, and washing with phosphate buffer; and
adding a CD45 secondary antibody solution labeled with fluorophore AlexaFluor 568, incubating at room temperature for 20 ~ 80 min, and washing with phosphate buffer.

4. The detection method according to any one of the preceding claims, is **characterized in that**, after incubated, all nucleated cells on the capture screen are labeled with nuclear fluorescent dye DAPI.

5. The detection method according to any one of the preceding claims, is **characterized in that**, the detection method further comprises the following step:
observing fluorescence color of esch channel to detect the tumor cell surface marker molecule PD-L1, through high-throughput multicolor imaging analysis using filters of CY5, FITC, PE and DAPI.

6. The detection method according to any of claims 1 to 5, is **characterized in that**, the mesh substrate has a size of 2 - 10 mm × 2 - 10 mm, and the screen has pores of 20 µm - 100 µm.

7. The detection method according to any of claims 1 to 5, is **characterized in that**, the mesh substrate comprises a stainless-steel body and a protective layer covering a surface of the stainless-steel body, the protective layer is made of a precious metal or an alloy thereof, and the EpCAM antibodies are arranged on the protective layer.

8. The detection method according to claim 7, is **characterized in that**, the protective layer is an AuPd layer deposited on the stainless-steel body by magnetron sputtering or electrochemical methods.

9. The detection method according to claim 7, is **characterized in that**, the EpCAM antibodies are attached to the protective layer via Traut's reagent or thiolate molecules with biotin-avidin.

10. The detection method according to any one of the preceding claims, is **characterized in that**, the sample to be detected is nucleated cells separated from body fluid.

11. The detection method according to claim 10, is **characterized in that**, the nucleated cells are obtained by separating peripheral blood using red blood cell lysing solution or lymphocyte separation solution.

12. The detection method according to claim 10, is **characterized in that**, after the nucleated cells flow through the capture screen and tumor cells bind to the capture screen, washing the capture screen with a cell washing solution to remove debris or cells that are not bound to the capture screen.

13. The detection method according to claim 10, is **characterized in that**, the body fluid is blood, urine or peritoneal fluid.

14. The detection method according to any one of the preceding claims, is **characterized in that**, the capture screen bound with the tumor cells is placed in a 4% paraformaldehyde
solution, maintained at room temperature for 10 ~ 60 min, and washed with phosphate buffer, to fix the captured tumor cells on the capture screen.

## Patentansprüche

1. Verfahren zum Nachweis des Tumorzell-Oberflächenmarker-Moleküls PD-L1, **dadurch gekennzeichnet, dass** das Verfahren folgende Schritte umfasst:
Bereitstellung eines Fanggitters, das mit Antikörpern versehen ist, die spezifisch an Tumorzellen binden können, wobei das Fanggitter ein Netzsubstrat und EpCAM-Antikörper, die auf dem Netzsubstrat durch Inkubation angeordnet sind, umfasst;
Durchleiten einer zu testenden Probe durch das Fanggitter, sodass Tumorzellen in der zu testenden Probe an das Fanggitter binden;
Fixierung der eingefangenen Tumorzellen auf dem Fanggitter; und
aufeinanderfolgende Verwendung einer primären PD-L1-Antikörperlösung, einer sekundären PD-L1-Antikörperlösung, die mit einem Fluorophor AlexaFluor 647 markiert ist, einer primären pan-CK-AlexaFluor 488-Antikörperlösung, einer primären CD45-Antikörperlösung und einer sekundären CD45-Antikörperlösung, die mit einem Fluorophor AlexaFluor 568 markiert ist, um die auf dem Fanggitter fixierten Zellen zu inkubieren, und anschließende Markierung aller Zellen auf dem Fanggitter mit einem kernhaltigen Fluoreszenzfarbstoff.

2. Nachweisverfahren nach Anspruch 1, **dadurch gekennzeichnet, dass** während der Inkubation das gesamte Fanggitter mit fixierten Tumorzellen als Träger für die Inkubation verwendet wird.

3. Nachweisverfahren nach Anspruch 1 oder 2, **dadurch gekennzeichnet, dass** die auf dem Fanggitter fixierten Zellen wie folgt inkubiert werden:
Zugabe einer primären PD-L1-Antikörperlösung, Inkubation der auf dem Fanggitter fixierten Zellen bei Raumtemperatur für 20 ~ 80 Minuten und anschließendes Waschen mit Phosphatpuffer;
Zugabe einer sekundären PD-L1-Antikörperlösung, die mit dem Fluorophor AlexaFluor 647 markiert ist, Inkubation bei Raumtemperatur für 20 ~ 80 Minuten und Waschen mit Phosphatpuffer;
Zugabe einer primären pan-CK-AlexaFluor 488-Antikörperlösung, Inkubation der auf dem Fanggitter fixierten Zellen bei Raumtemperatur für 20 ~ 80 Minuten und Waschen mit Phosphatpuffer;
Zugabe einer primären CD45-Antikörperlösung, Inkubation der auf dem Fanggitter fixierten Zellen bei Raumtemperatur für 20 ~ 80 Minuten und Waschen mit Phosphatpuffer; und
Zugabe einer sekundären CD45-Antikörperlösung, die mit dem Fluorophor AlexaFluor 568 markiert ist, Inkubation bei Raumtemperatur für 20 ~ 80 Minuten und Waschen mit Phosphatpuffer.

4. Nachweisverfahren nach einem der vorhergehenden Ansprüche, **dadurch gekennzeichnet, dass** nach der Inkubation alle kernhaltigen Zellen auf dem Fanggitter mit dem kernhaltigen Fluoreszenzfarbstoff DAPI markiert werden.

5. Nachweisverfahren nach einem der vorhergehenden Ansprüche, **dadurch gekennzeichnet, dass** das Nachweisverfahren weiterhin den folgenden Schritt umfasst:
Beobachtung der Fluoreszenzfarbe jedes Kanals zum Nachweis des Tumorzell-Oberflächenmarker-Moleküls PD-L1 durch Hochdurchsatz-Multicolor-Imaging-Analyse unter Verwendung von Filtern für CY5, FITC, PE und DAPI.

6. Nachweisverfahren nach einem der Ansprüche 1 bis 5, **dadurch gekennzeichnet, dass** das Netzsubstrat eine Größe von 2 - 10 mm × 2 - 10 mm hat und das Gitter Poren von 20 µm - 100 µm aufweist.

7. Nachweisverfahren nach einem der Ansprüche 1 bis 5, **dadurch gekennzeichnet, dass** das Netzsubstrat einen Edelstahlkörper und eine eine Oberfläche des Edelstahlkörpers bedeckende Schutzschicht umfasst, die Schutzschicht aus einem Edelmetall oder einer Legierung davon besteht und die EpCAM-Antikörper auf der Schutzschicht angeordnet sind.

8. Nachweisverfahren nach Anspruch 7, **dadurch gekennzeichnet, dass** die Schutzschicht eine AuPd-Schicht ist, die durch Magnetron-Sputtern oder elektrochemische Methoden auf den Edelstahlkörper aufgetragen wird.

9. Nachweisverfahren nach Anspruch 7, **dadurch gekennzeichnet, dass** die EpCAM-Antikörper mittels Trauts-Reagenz oder thiolhaltiger Moleküle mit Biotin-Avidin an die Schutzschicht gebunden werden.

10. Nachweisverfahren nach einem der vorhergehenden Ansprüche, **dadurch gekennzeichnet, dass** es sich bei der nachzuweisenden Probe um kernhaltige Zellen handelt, die von Körperflüssigkeit abgetrennt wurden.

11. Nachweisverfahren nach Anspruch 10, **dadurch gekennzeichnet, dass** die kernhaltigen Zellen durch Trennung von peripherem Blut unter Verwendung einer Erythrozyten-Lyse-Lösung oder einer Lymphozyten-Trennlösung gewonnen werden.

12. Nachweisverfahren nach Anspruch 10, **dadurch gekennzeichnet, dass** nach dem Hindurchleiten der kernhaltigen Zellen durch das Fanggitter und dem Binden der Tumorzellen an das Fanggitter Waschen des Fanggitters mit einer Zellwaschlösung, um Schmutz oder Zellen, die nicht an das Fanggitter gebunden sind, zu entfernen.

13. Nachweisverfahren nach Anspruch 10, **dadurch gekennzeichnet, dass** die Körperflüssigkeit Blut, Urin oder Peritonealflüssigkeit ist.

14. Nachweisverfahren nach einem der vorhergehenden Ansprüche, **dadurch gekennzeichnet, dass** das mit den Tumorzellen gebundene Fanggitter in eine 4%ige Paraformaldehyd-Lösung gegeben, bei Raumtemperatur 10 ~ 60 Minuten stehen gelassen und mit Phosphatpuffer gewaschen wird, um die eingefangenen Tumorzellen auf dem Fanggitter zu fixieren.

## Revendications

1. Méthode de détection d'une molécule marqueur de surface des cellules tumorales PD-L1, **caractérisée par le fait que** la méthode comprend les étapes suivantes :
fournir un écran de capture contenant des anticorps capables de se lier spécifiquement à des cellules tumorales, dans lequel l'écran de capture comprend un substrat en maille et des anticorps EpCAM disposés sur le substrat en maille par incubation ;
faire circuler un échantillon à tester à travers l'écran de capture, de sorte que les cellules tumorales de l'échantillon à tester se lient à l'écran de capture ;
fixer les cellules tumorales capturées sur l'écran de capture ; et
utiliser successivement une solution d'anticorps primaire PD-L1, une solution d'anticorps secondaire PD-L1 marquée avec un fluorophore AlexaFluor 647, une solution d'anticorps primaire pan-CK-AlexaFluor 488, une solution d'anticorps primaire CD45 et une solution d'anticorps secondaire CD45 marquée avec un fluorophore AlexaFluor 568, pour incuber les cellules fixées sur l'écran de capture, et ensuite marquer toutes les cellules sur l'écran de capture avec un colorant fluorescent nucléaire.

2. La méthode de détection selon la revendication 1, est **caractérisée par le fait que**, pendant l'incubation, l'écran de capture entier avec les cellules tumorales fixées est utilisé comme support pour l'incubation.

3. La méthode de détection selon la revendication 1 ou 2 est **caractérisée par le fait que** les cellules fixées sur l'écran de capture sont incubées comme suit :
ajout d'une solution d'anticorps primaire PD-L1, incubation des cellules fixées sur l'écran de capture à température ambiante pendant 20 ~ 80 min, puis lavage avec un tampon phosphate ;
ajout d'une solution d'anticorps secondaire PD-L1 marqué avec le fluorophore AlexaFluor 647, incubation à température ambiante pendant 20 ~ 80 min, et lavage avec un tampon phosphate ;
ajout d'une solution d'anticorps primaire pan-CK-AlexaFluor 488, incubation des cellules fixées sur l'écran de capture à température ambiante pendant 20 ~ 80 min, et lavage avec un tampon phosphate ;
ajout d'une solution d'anticorps primaire CD45, incubation des cellules fixées sur l'écran de capture à température ambiante pendant 20 à 80 minutes, et lavage avec un tampon phosphate ; et
ajout d'une solution d'anticorps secondaire CD45 marqué avec le fluorophore AlexaFluor 568, incubation à température ambiante pendant 20 ~ 80 min, et lavage avec un tampon phosphate.

4. La méthode de détection selon l'une quelconque des revendications précédentes, est **caractérisée par le fait que**, après incubation, toutes les cellules nucléées sur l'écran de capture sont marquées avec le colorant fluorescent nucléaire DAPI.

5. La méthode de détection selon l'une quelconque des revendications précédentes, est **caractérisée en ce que** la méthode de détection comprend en outre l'étape suivante : observation de la couleur de fluorescence de chaque canal pour détecter la molécule PD-L1, marqueur de la surface des cellules tumorales, par une analyse d'imagerie multicolore à haut débit utilisant des filtres CY5, FITC, PE et DAPI.

6. La méthode de détection selon l'une des revendications 1 à 5, est **caractérisée en ce que**, le substrat maillé a une taille de 2 - 10 mm × 2 - 10 mm, et l'écran a des pores de 20 µm - 100 µm.

7. Le procédé de détection selon l'une quelconque des revendications 1 à 5, est **caractérisé en ce que**, le substrat maillé comprend un corps en acier inoxydable et une couche protectrice recouvrant une surface du corps en acier inoxydable, la couche protectrice est constituée d'un métal précieux ou d'un de ses alliages, et les anticorps EpCAM sont disposés sur la couche protectrice.

8. La méthode de détection selon la revendication 7, est **caractérisée par le fait que** la couche protectrice est une couche d'AuPd déposée sur le corps en acier inoxydable par pulvérisation cathodique ou par des méthodes électrochimiques.

9. La méthode de détection selon la revendication 7, est **caractérisée par le fait que** les anticorps EpCAM sont fixés à la couche protectrice par l'intermédiaire du réactif de Traut ou de molécules de thiolate avec de la biotine-avidine.

10. La méthode de détection selon l'une quelconque des revendications précédentes, est **caractérisée par le fait que** l'échantillon à détecter est constitué de cellules nucléées séparées du fluide corporel.

11. La méthode de détection selon la revendication 10, est **caractérisée par le fait que** les cellules nucléées sont obtenues par séparation du sang périphérique à l'aide d'une solution de lyse des globules rouges ou d'une solution de séparation des lymphocytes.

12. La méthode de détection selon la revendication 10, est **caractérisée en ce que**, après que les cellules nucléées ont traversé l'écran de capture et que les cellules tumorales se sont liées à l'écran de capture, le lavage de l'écran de capture avec une solution de lavage cellulaire pour éliminer les débris ou les cellules qui ne sont pas liées à l'écran de capture.

13. La méthode de détection selon la revendication 10, est **caractérisée par le fait que** le liquide corporel est du sang, de l'urine ou du liquide péritonéal.

14. La méthode de détection selon l'une quelconque des revendications précédentes est **caractérisée par le fait que** l'écran de capture lié aux cellules tumorales est placé dans une solution de paraformaldéhyde à 4 %, maintenu à température ambiante pendant 10 ~ 60 mm, et lavé avec un tampon phosphate, pour fixer les cellules tumorales capturées sur l'écran de capture.
